# EUROPÄISCHE PATENTANMELDUNG

(11) **EP 0 683 212 A1**
(43) Veröffentlichungstag der Anmeldung: **22.11.1995**
(21) Anmeldenummer: 95106924.4
(22) Anmeldetag: 08.05.1995
(51) Int. Cl.: C09B 57/12, C07D 471/16, C07D 487/16, C08K 5/3465

(54) **Neue Farbstoffe zum Massefärben von Kunststoffen**

(30) Priorität: 20.05.1994 DE 4417746
(71) Anmelder: BAYER AG, 51368 Leverkusen (DE)
(72) Erfinder: Roschger, Peter, Dr., D-51063 Köln (DE); Hederich, Volker, Dr., D-51061 Köln (DE); Michaelis, Stephan, Dr., D-51375 Leverkusen (DE); Kröck, Friedrich Wilhelm, Dr., D-51519 Odenthal (DE)

(57) **Zusammenfassung**

Es wurden Farbstoffe der Formel 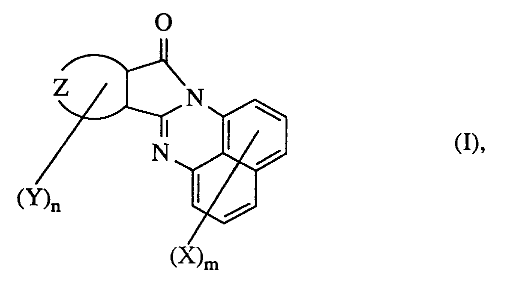
worin
- Z: ein Rest zur Vervollständigung eines 3,4- oder 2,3-Pyridinylen-, eines 4,5-Pyrmidinylen-, eines 3,4- oder 4,5-Pyridazinylen- oder eines 2,3-Pyrazinylen-Systems bedeutet und
- X, Y, m und n: die in der Beschreibung genannten Bedeutungen besitzen,
gefunden, die sich besonders gut zum Massefärben von Kunststoffen, insbesondere von Thermoplasten, eignen.

## Beschreibung

Die Erfindung betrifft Farbstoffe, ein Verfahren zu ihrer Herstellung und ihre Verwendung zum Massefärben von Kunststoffen.

Farbstoffe, wie sie z.B. in JP-A-4 909 530, JP-A-4 909 552, FR 1.166.701 und FR 1.075.110 beschrieben sind, sind zum Färben von Kunststoffen bekannt, weisen aber im Hinblick auf deren Echtheiten gewisse Nachteile auf.

Nun wurden Azaphthaloperinonfarbstoffe der allgemeinen Formel (I) gefunden 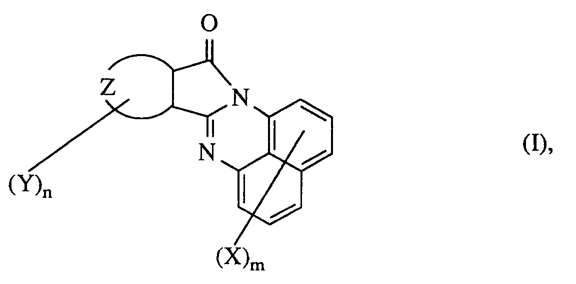
worin
- Z: ein Rest zur Vervollständigung eines 3,4- oder 2,3-Pyridinylen-, eines 4,5-Pyrimidinylen-, eines 3,4- oder 4,5-Pyridazinylen- oder eines 2,3-Pyrazinylen-Systems bedeutet,
- X: Alkyl, Halogen, Nitro, Chlorsulfonyl, Aryloxysulfonyl, Hydroxy, Alkoxy, Acyloxy, ein gegebenenfalls durch Alkyl oder Aryl substituiertes Aminosulfonyl, oder ein ankondensierter cycloaliphatischer oder heterocyclischer Ring ist,
- Y: Alkyl, Aryl, Halogen, Nitro, Hydroxy, Alkoxy, Chlorsulfonyl, Acyloxy, insbesondere Aryloxysulfonyl, eine gegebenenfalls durch Acyl oder Alkyl substituierte Aminogruppe, ein gegebenenfalls durch Alkyl oder Aryl substituierter Aminosulfonylrest oder ein ankondensierter cycloaliphatischer oder aromatischer Ring ist,
- m: eine Zahl zwischen 0 und 6,
- n: eine Zahl zwischen 0 und 3 ist
und für m > 1
X jeweils verschiedene oder gleiche der oben genannten Bedeutungen haben kann,
und für n > 1
Y jeweils verschiedene oder gleiche der oben genannten Bedeutungen haben kann.

Unter Alkyl bzw. Alkoxy wird vorzugsweise C₁-C₁₈-Alkyl, insbesondere C₁-C₆-Alkyl bzw. Alkoxy verstanden, unter Aryl wird vorzugsweise C₆-C₁₄, insbesondere C₆-C₁₀; unter Halogen vorzugsweise Cl, Br oder F verstanden und unter Acyl vorzugsweise C₁-C₁₈, insbesondere C₁-C₆-Alkylcarbonyl, -sulfonyl oder C₆-C₁₄, insbesondere C₆-C₁₀-Arylcarbonyl,-sulfonyl verstanden.

Bevorzugt sind Farbstoffe der Formel I, worin
- Z: ein Rest zur Vervollständigung eines 3,4-Pyridinylensystems bedeutet.

In einer besonderen Ausführungsform bedeuten
- X: Chlor, Brom, -NO₂, -OCH₃, -OCH₂(C₆H₅), Chlorsulfonyl, -OH, -SO₂O(C₆H₅), -SO₂N(CH₃)₂, -SO₂NHCH₃, Methyl, Ethyl, n-Propyl, Isopropyl, n-Butyl, iso-Butyl, sek.-Butyl, tert.-Butyl oder ein vorzugsweise in 4,5-Stellung ankondensierter cycloaliphatischer 5- oder 6-Ring, wobei -C₆H₅ hier und auch im folgenden für Phenyl steht,
- Y: Methyl, Ethyl, n-Propyl, Isopropyl, n-Butyl, iso-Butyl, sek.-Butyl, tert.-Butyl, ein gegebenenfalls durch C₁-C₆-Alkoxy, C₁-C₆-Alkyl, Cl, Br oder F substituiertes Phenyl, ein ankondensierter cycloaliphatischer 5-, 6- oder 7-Ring oder ein ankondensierter Benzo-Ring, der gegebenenfalls durch Cl, Br, C₁-C₄-Alkyl oder C₁-C₄-Alkoxy substituiert ist,
- m: eine Zahl zwischen 0 und 4 und
- n: eine Zahl zwischen 0 und 3.

In einer ganz besonders bevorzugten Ausführungsform bedeutet m gleich 0.

Unter der allgemeinen Formel (I) der erfindungsgemäßen Farbstoffe werden entweder isomerenreine Verbindungen oder Mischungen davon verstanden. Bevorzugte Farbstoffe der Formel (I) entsprechen den Formeln Ia und Ib.

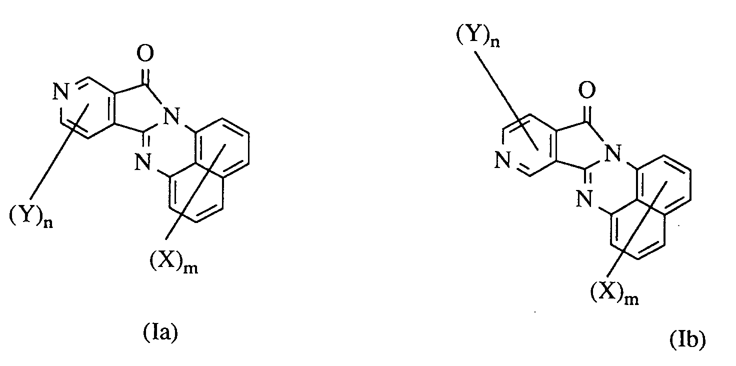

Die Indices n und m können dabei den Charakter einer statistischen Angabe haben und somit eine beliebige Zahl des definierten Bereiches annehmen.

Die erfindungsgemäßen Farbstoffe der Formel (I) lassen sich nach an sich bekannten Verfahren (vgl. z.B. D.R.P 202 354, Chem. Ber. 75 (1942), 719; Liebigs Ann. Chem. 365 (1909), 128) durch Kondensation von Dicarbonsäuren bzw. deren funktionellen Derivaten der Formel (II) 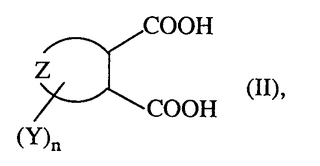
worin Z, Y und n die obigen Bedeutungen haben,
mit Naphthalin-1,8-diaminen der Formel (III) 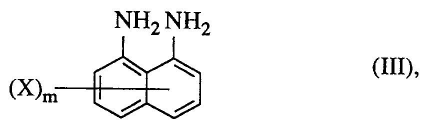
in welcher

- X und m: die angeführten Bedeutungen haben,
darstellen.

Insbesondere werden bei diesem Verfahren Dicarbonsäuren bzw. deren funktionelle Derivate der Formel (IIa) - (IIf) eingesetzt, 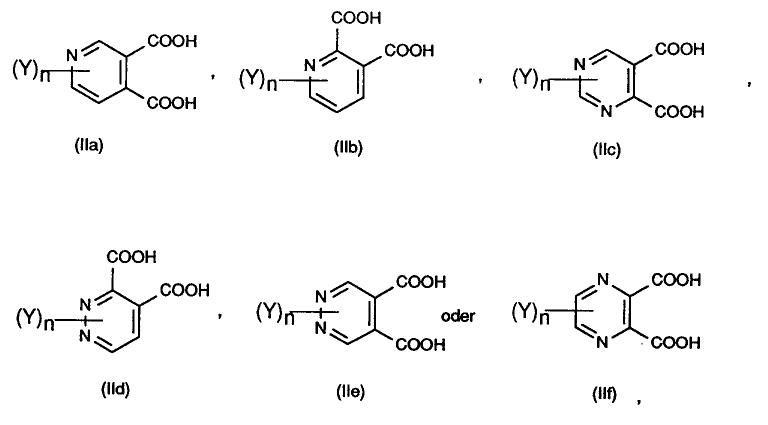
worin
- Y und n: die genannten Bedeutungen besitzen.

Besonders bevorzugt ist der Einsatz von Dicarbonsäuren der Formel (IIa) bzw. deren funktionelle Derivate, insbesondere deren Anhydride.

Bevorzugte funktionelle Derivate der Dicarbonsäuren sind deren Anhydride.

Die Kondensation kann dabei direkt durch Zusammenschmelzen äquimolarer Mengen der Komponenten der Formeln (II) und (III) bei Temperaturen zwischen 50°C und 220°C, vorzugsweise bei 120 bis 180°C, erfolgen, oder vorteilhafter in einem Lösungsmittel bei Temperaturen zwischen 20°C und 220°C, vorzugsweise 50 bis 180°C, gegebenenfalls unter Druck durchgeführt werden, wobei eine destillative Entfernung des Reaktionswassers erfolgen kann.

Geeignete Lösungsmittel sind beispielsweise: Chlorbenzol, o-Dichlorbenzol, Trichlorbenzol, Xylol, Dimethylformamid, N-Methylpyrrolidon, Eisessig, Propionsäure, Phenol, Kresole, Phenoxyethanol, Glykole und deren Mono- und Dialkylether, Alkohole, z.B. Methanol, Ethanol, i-Propanol, Wasser und wäßrige Lösungsmittel wie z.B. verdünnte Schwefelsäure u.a..

Gegebenenfalls kann die Reaktion unter Zusatz eines sauren Katalysators erfolgen.

Geeignete Katalysatoren sind beispielsweise: Zinkchlorid, p-Toluolsulfonsäure, Salzsäure, Schwefelsäure, organische Säuren u.a..

Die entsprechenden Pyridin-3,4-dicarbonsäuren (bzw. deren funktionelle Derivate) sind im Handel erhältlich oder lassen sich z.B. nach Chem. Ber. 50, S. 1568-1584 (1917) darstellen. Geeignete Pyridindicarbonsäuren sind beispielsweise:

Pyridin-3,4-dicarbonsäure, 2,6-Dimethylpyridin-3,4-dicarbonsäure, 2,5,6-Trimethyl-pyridin-3,4-dicarbonsäure, 6-Phenyl-pyridin-3,4-dicarbonsäure, 6-Phenyl-2-methyl-pyridin-3,4-dicarbonsäure, 5,6-Tetramethylen-pyridin-3,4-dicarbonsäure, 5,6-Trimethylen-pyridin-3,4-dicarbonsäure, 6-(4-Chlorphenyl-)-pyridin-3,4-dicarbonsäure, 6-(2,4-Dichlorphenyl-)-2-methyl-pyridin-3,4-dicarbonsäure, 2,6-Diphenyl-pyridin-3,4-dicarbonsäure, 5,6-Diphenyl-pyridin-3,4-dicarbonsäure, 2,6-Diethyl-pyridin-3,4-dicarbonsäure, 2-Ethyl-5-methyl-6-pheny-pyridin-3,4-dicarbonsäure, Chinolin-3,4-dicarbonsäure, 2-Methylchinolin-3,4-carbonsäure, 5,6,7,8-Tetrachlor-2-methyl-chinolin-3,4-dicarbonsäure, Chlor- und Brom-2-ethylchinolin-3,4-dicarbonsäure, Dimethyl- und Trimethyl-chinolin-3,4-dicarbonsäure, Benzochinolin-3,4-dicarbonsäure, Pyridin-2,3-dicarbonsäure, Chinolin-2,3-dicarbonsäure oder deren Anhydride.

Die eingesetzten substituierten Naphthalin-1,8-diamine sind im Handel erhältlich oder lassen sich z.B. nach D.R.P 122 475; D.R.P 108 166; u.a. oder in Analogie dazu herstellen. Geeignete Naphthalin-1,8-diamine sind beispielsweise: 1,8-Napthalindiamin, Chlor-1,8-naphthalindiamine, Dichlor-1,8-naphthalindiamine, Brom-1,8-naphthalindiamine, Methyl-1,8-naphthalindiamine, Dimethyl-1,8-naphthalindiamine, Methyl-chlor-1,8-naphthalindiamine, Methoxy-1,8-naphthalindiamine, Ethoxy-1,8-naphthalindiamine, Acetamino-1,8-naphthalindiamine und 4,5-Dimethylennaphthalin-1,8-diamin, wobei die Substituenten vorzugsweise in 2-, 4-, 5- und 7-Stellung des Naphthalins gebunden sind.

Die erfindungsgemäßen Farbstoffe der Formel (I) können demnach aus unsubstituierten und/oder substituierten Edukten mit einem sich gegebenenfalls anschließenden Substituentenaustausch hergestellt werden. Unter Substituentenaustausch wird dabei sowohl der Austausch eines Wasserstoffs oder anderer Liganden durch einen Substituenten, z.B. mittels Chlorierung, Bromierung, Sulfonierung, Chlorsulfonierung oder Nitrierung als auch die Modifizierung von Substituenten verstanden, wie im folgenden beispielhaft ausgeführt wird.

Farbstoffe der Formel (I), in denen X und/oder Y für einen Alkyl- oder Arylaminosulfonylrest stehen, lassen sich außer nach dem erstgenannten Verfahren auch aus den entsprechenden Farbstoffen der Formel (I), in denen X und/oder Y einen Chlorsulfonylrest bedeuten, mit Alkyl- bzw. Arylaminen darstellen.

Erfindungsgemäße Farbstoffe, in denen X und/oder Y gleich einem Aryloxysulfonylrest sind, können auch durch Umsetzung der entsprechenden Chlorsulfonyl-Farbstoffe mit Phenolen oder Naphtholen in Gegenwart einer Base, z.B. Pyridin, Triethylamin, Alkali- bzw. Erdalkalicarbonaten, -hydroxiden oder -oxiden gewonnen werden.

Farbstoffe der Formel (I), in denen X und/oder Y für Alkyloxy oder Acyloxy stehen, können zusätzlich durch Alkylierung bzw. Acylierung der erfindungsgemäßen Farbstoffe, in welchen X und/oder Y eine Hydroxygruppe bedeuten, dargestellt werden.

Jene Farbstoffe der Formel (I) mit X und/oder Y gleich einer gegebenenfalls acylierten bzw. alkylierten Aminogruppe können außerdem durch Reduktion mit üblichen Reduktionsmitteln, z.B. Eisen, Zink, Natriumsulfid, Wasserstoff u.a. der entsprechenden Verbindungen, in denen X und/oder Y für eine Nitrogruppe stehen, und gegebenenfalls nachfolgende Acylierung bzw. Alkylierung gewonnen werden. Der Acylierungsschritt kann auch im Zuge der Reduktion durch Zusatz eines Acylierungsmittels wie Carbonsäureanhydriden erfolgen.

Die erfindungsgemäßen Farbstoffe eignen sich hervorragend zum Färben von Kunststoffen in der Masse.

Die Erfindung betrifft daher weiterhin ein Verfahren zum Färben von Kunststoffen in der Masse.

Unter Massefärben wird hierbei insbesondere ein Verfahren verstanden, bei dem der Farbstoff in die geschmolzene Kunststoffmasse eingearbeitet wird, z.B. unter Zuhilfenahme eines Extruders, oder bei dem der Farbstoff bereits Ausgangskomponenten zur Herstellung des Kunststoffes, z.B. Monomeren vor der Polymerisation, zugesetzt wird.

Besonders bevorzugte Kunststoffe sind Thermoplaste, beispielsweise Vinylpolymere, Celluloseester, Polyester und Polyamide.

Geeignete Vinylpolymere sind Polystyrol, Styrol-Acrylnitril-Copolymere, Styrol-Butadien-Copolymere, Styrol-Butadien-Acrylnitril-Terpolymere, Polymethylmethacrylat u.a.

Weiterhin geeignete Polyester sind: Polyalkylenterephthalate wie Polyethylenterephthalate und Polycarbonate.

Bevorzugt sind Polystyrol, Styrol-Mischpolymere, Polycarbonate und Polymethacrylat. Besonders bevorzugt ist Polystyrol.

Die erwähnten hochmolekularen Verbindungen können einzeln oder in Gemischen, als plastische Massen oder Schmelzen vorliegen.

Die erfindungsgemäßen Farbstoffe werden in feinverteilter Form zur Anwendung gebracht, wobei Dispergiermittel mitverwendet werden können, aber nicht müssen.

Werden die Farbstoffe (I) nach der Polymerisation eingesetzt, so werden sie mit dem Kunststoffgranulat trocken vermischt oder vermahlen und dieses Gemisch z.B. auf Mischwalzen oder in Schnecken plastifiziert und homogenisiert. Man kann die Farbstoffe aber auch der schmelzflüssigen Masse zugeben und diese durch Rühren homogen verteilen. Das derart vorgefärbte Material wird dann wie üblich, z.B. durch Verspinnen zu Borsten, Fäden usw. oder durch Extrusion oder im Spritzguß-Verfahren, zu Formteilen weiterverarbeitet.

Die Erfindung betrifft daher auch die Kunststoffe, die nach einem der vorgenannten Verfahren gefärbt wurden. Dazu zählen beispielsweise Borsten, Fäden, Formteile oder Folien.

Da die Farbstoffe der Formel (I) gegenüber Polymerisationskatalysatoren, insbesondere Peroxiden, beständig sind, ist es auch möglich, die Farbstoffe den monomeren Ausgangsmaterialien für die Kunststoffe zuzusetzen und dann in Gegenwart von Polymerisationskatalysatoren zu polymerisieren. Dazu werden die Farbstoffe vorzugsweise in den monomeren Komponenten gelöst oder mit ihnen innig vermischt.

Die Farbstoffe der Formel (I) werden vorzugsweise zum Färben der genannten Polymeren in Mengen von 0,0001 bis 1 Gew.-%, insbesondere 0,01 bis 0,5 Gew.-%, bezogen auf die Polymermenge, eingesetzt.

Durch Zusatz von in den Polymeren unlöslichen Pigmenten, wie z.B. Titandioxid, können entsprechende wertvolle gedeckte Färbungen erhalten werden.

Titandioxid kann in einer Menge von 0,01 bis 10 Gew.-%, vorzugsweise 0,1 bis 5 Gew.-%, bezogen auf die Polymermenge, verwendet werden.

Nach dem erfindungsgemäßen Verfahren erhält man transparente bzw. gedeckte brillante, orange bis violette Färbungen mit guter Hitzebeständigkeit sowie guter Licht- und Wetterechtheit.

In das erfindungsgemäße Verfahren können auch Mischungen verschiedener Farbstoffe der Formel (I) und/oder Mischungen von Farbstoffen der Formel (I) mit anderen Farbstoffen und/oder anorganischen bzw. organischen Pigmenten eingesetzt werden.

Die Erfindung wird erläutert durch, jedoch nicht beschränkt auf die folgenden Beispiele, in denen die Teile gewichtsmäßig angegeben sind und Prozentangaben Gewichtsprozente (Gew.-%) bedeuten.

### Beispiel 1

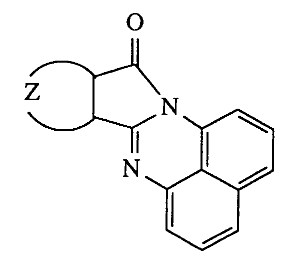

(Z = Rest zur Vervollständigung eines 2,6-Dimethyl-3,4-pyridinylen-Ringes)

### A) Herstellung

Eine Mischung aus 4,8 g Naphthalin-1,8-diamin, 4,8 g 2,6-Dimethyl-pyridin-3,4-dicarbonsäure und 35 g Phenol wird unter Stickstoff 1 Stunde auf 150°C erhitzt. Anschließend kühlt man auf 80°C, verdünnt mit 30 ml Methanol und saugt den gebildeten Niederschlag nach dem Erkalten ab. Nach dem Waschen mit Methanol und Trocknen erhält man 6,0 g des Farbstoffes der obigen Formel.

### B) Färbebeispiele

### Beispiel a)

100 Teile Polystyrol-Granulat und 0,02 Teile eines Farbstoffes der obigen Formel werden im Trommelmischer während 15 Minuten intensiv vermischt. Das trocken angefärbte Granulat wird bei 240°C auf einer Schneckenspritzgießmaschine verarbeitet. Man erhält transparente, rote Platten von sehr guter Lichtechtheit. Anstelle von Polystyrol-Polymerisat können auch Butadien/Acrylnitril-Mischpolymerisate wie Styrol-Butadien-Acrylnitril verwendet werden. Setzt man zusätzlich 0,5 Teile Titandioxid zu, so erhält man farbstarke gedeckte Färbungen.

### Beispiel b)

0,015 Teile des Farbstoffes aus Beispiel A) und 100 Teile Polymethylmethacrylat werden trocken vermischt und auf einem 1-Wellenextruder bei 230°C homogenisiert. Das als Strang aus dem Extruder austretende Material wird granuliert. Es kann anschließend zu Formen verpreßt werden. Man erhält einen transparent rot-gefärbten Kunststoff mit guter Licht- und Wetterechtheit.

### Beispiel c)

100 Teile eines handelsüblichen Polycarbonats werden in Form von Granulat mit 0,03 Teilen des Farbstoffes aus Beispiel A) trocken gemischt. Das so bestäubte Granulat wird auf einem 2-Wellenextruder bei 290°C homogenisiert. Man erhält eine transparente rote Färbung von guter Lichtechtheit. Das gefärbte Polycarbonat wird als Strang aus dem Extruder ausgetragen und zu Granulat verarbeitet. Das Granulat kann nach üblichen Methoden verarbeitet werden. Arbeitet man wie oben beschrieben, aber unter Zusatz von 1 % Titandioxid, so erhält man eine rote gedeckte Färbung.

### Beispiel d)

Mit 100 Teilen Styrol-Acrylnitril-Copolymerisat werden 0,04 Teile des Farbstoffes aus Beispiel A) trocken vermischt und in einem 2-Wellenextruder bei 190°C homogenisiert, granuliert und dann zu Formen auf übliche Weise verpreßt. Man erhält einen transparent roten Kunststoff von guter Lichtechtheit.

### Beispiel e)

0,025 Teile des Farbstoffes aus Beispiel A) werden mit 100 Teilen Polyethylenterephthalat einer transparenten Type vermischt und in einem 2-Wellenextruder bei 280°C homogenisiert. Man erhält eine transparente, rote Färbung mit guter Lichtechtheit. Nach anschließender Granulierung kann der eingefärbte Kunststoff nach den üblichen Methoden der thermoplastischen Verformung verarbeitet werden. Arbeitet man unter Zusatz von 1 % Titandioxid, so erhält man eine gedeckte Färbung.

### Beispiel f)

In 98,9 Teilen Styrol werden 0,05 Teile tert.-Dodecylmercaptan sowie 0,05 Teile des Farbstoffes aus Beispiel A) gelöst. Diese Lösung dispergiert man in einer Lösung aus 200 Teilen entsalztem Wasser, 0,3 Teilen teilverseiftem Polyvinylacetat (z.B. Mowiol® 50/88 der Fa. Hoechst) und 0,05 Teilen Dodecylbenzolsulfonat. Nach Zusatz von 0,1 Teilen Dibenzoylperoxid in 1 Teil Styrol wird die Dispersion unter kräftigem Rühren auf 80°C erhitzt und die Polymerisation gestartet. Bei Anwendung folgender Polymerisationsbedingungen: 4 h bei 80°C, 2 h bei 90°C, 3 h bei 110°C, 2 h bei 130°C, erhält man das Polymerisat in einer Ausbeute von 98 % der Theorie. Das Polymerisat fällt in Form von Perlen an, die je nach Rührbedingungen einen Durchmesser von 0,1 bis 1,5 mm (D₅₀-Wert) aufweisen. Das Polymerisat wird durch Filtration vom Serum getrennt, bei 110°C auf eine Restfeuchte von 0,5 % getrocknet. Nach dem Aufschmelzen in einem Mischaggregat (Heißwalze) werden 0,5 % Zinkstearat und 0,2 % Ionol zugemischt und das Polymerisat granuliert.

Das Polymerisat kann nach den üblichen Methoden der thermoplastischen Verformung, z.B. im Spritzguß-Verfahren, zu roten, transparenten Formteilen verarbeitet werden.

### Beispiel g)

In 74,8 Teilen Styrol und 25 Teilen Acrylnitril werden 0,2 Teile tert.-Dodecylmercaptan sowie 0,01 Teile des Farbstoffes aus Beispiel A) gelöst, diese Lösung wird anschließend in einer Lösung aus 200 Teilen vollentsalztem Wasser und 0,2 Teilen eines mit Natriumhydroxid neutralisierten Copolymerisats von Styrol und Maleinsäureanhydrid dispergiert. Nach Zusatz von 0,1 Teilen Dibenzoylperoxid, gelöst in einem Teil Styrol, wird die Dispersion unter kräftigem Rühren auf 80°C erhitzt und die Polymerisation gestartet. Nach der Polymerisation wie im Beispiel f) wird auch in gleicher Weise, wie im Beispiel angegeben, aufgearbeitet. Als Schmiermittel wird 0,5 % Zinkstearat und als Alterungsschutzmittel 0,5 % Ionol auf der Heißwalze eingearbeitet. Das granulierte Polymerisat läßt sich zu transparenten roten Formteilen verspritzen.

### Beispiel h)

In einem kontinuierlich arbeitenden, überflutet gefahrenen Vorreaktor wird eine Lösung aus 99,95 Teilen Styrol, 0,04 Teilen des Farbstoffes aus Beispiel A) und 0,01 Teilen Di-tert.-butylperoxid eingefahren und bei einer Temperatur von 75°C anpolymerisiert. Die aus dem Vorteaktor austretende, anpolymerisierte Lösung (Polystyrol-Gehalt 20 %) wird in einem 2-Wellenschneckenaggregat eingeführt. Die beiden Wellen laufen mit 20 Upm gegenläufig. Die vier heiz- bzw. kühlbaren Segmente der Schneckenmaschine werden in der Reihenfolge Produkteintritt - Produktaustritt auf 110°C, 130°C, 160°C, 180°C gehalten. Das Polymerisat verläßt den Schneckenreaktor mit einer Feststoffkonzentration von 80 %. In einem nachgeschalteten Extruder werden 3 Gew.-Teile Ionol und 5 Gew.-Teile Octylalkohol pro 1000 Gew.-Teile Polymerlösung zudosiert, das Polymerisat entgast und anschließend granuliert. Das rot gefärbte Granulat kann zu Formteilen verarbeitet werden.

### Beispiel i)

0,02 Teile des Farbstoffes aus Beispiel A) werden in 74,97 Teilen Styrol und 25 Teilen Acrylnitril bzw. Methacrylnitril gelöst. Nach Zugabe von 0,01 Teilen Di-tert.-butylperoxid wird die so erhaltene Lösung in einem kontinuierlich arbeitenden, überflutet gefahrenen Vorteaktor eingefahren. Die Polymerisation und Aufarbeitung erfolgt wie im Beispiel h) angegeben. Das transparente, rote Granulat kann nach den üblichen Methoden der Verarbeitung thermoplastischer Massen zu Profilen und Platten weiterverarbeitet werden.

### Beispiel k)

In 99,97 Teilen Methylmethacrylat werden 0,03 Teile des Farbstoffes aus Beispiel A) gelöst. Nach Zugabe von 0,1 Teilen Dibenzoylperoxid wird die Lösung auf 120°C erhitzt und die Polymerisation gestartet. Nach 30 Min. wird das anpolymerisierte Methylmethacrylat zwischen zwei Glasplatten bei 80°C während zehn Stunden auspolymerisiert. Man erhält rote, transparente Polymethylmethacrylat-Platten.

### Beispiel l)

100 Teile Polyamid-6-Schnitzel, erhalten durch Polymerisation von ε-Caprolactam, werden mit 0,05 Teilen des Farbstoffes aus Beispiel A) in einer Schüttelmaschine innig vermischt. Die so erhaltenen gepuderten Schnitzel werden bei 260°C in einem Extruder aufgeschmolzen, die erhaltene Schmelze durch eine Einloch-Düse vom Durchmesser 0,5 mm gepreßt und der austretende Faden mit einer Geschwindigkeit von ca. 25 m/Min. abgezogen. Der Faden läßt sich in heißem Wasser auf das Vierfache verstrecken. Erhalten wird ein transparent rot gefärbter Faden von ausgezeichneter Lichtechtheit. Will man eine gedeckte Färbung erhalten, so gibt man zusätzlich 0,5 Teile Titandioxid zu.

Die Verweilzeit im Extruder kann ohne Beeinträchtigung des Farbtons bis zu 30 Minuten betragen.

### Beispiel 2

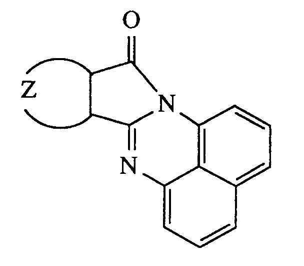

(Z = Rest zur Vervollständigung eines 3,4-Pyridinylen-Ringes)
4,8 g Naphthalin-1,8-diamin und 5,0 g Pyridin-3,4-dicarbonsäure werden in 50 ml Pyridin bei 70°C gelöst und anschließend mit 10 ml Acetanhydrid versetzt, wobei die Temperatur auf 120°C steigt. Man erhitzt noch 30 Min zum Rückfluß und läßt dann erkalten. Der Niederschlag wird abgesaugt und erst mit wenig Pyridin, dann mit Methanol gewaschen und schließlich getrocknet. Ausbeute: 5,5 g
Zur Reinigung kann der Farbstoff aus Toluol umkristallisiert werden.

Der Farbstoff ergibt in Kunststoffen, analog zu Färbebeispielen B) in Beispiel 1 gefärbt, gelbstichig rote Färbungen mit hohem Echtheitsniveau.

### Beispiel 3

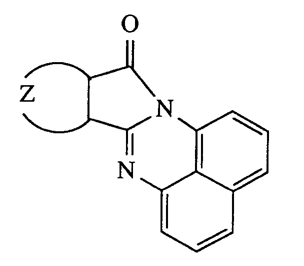

(Z = Rest zur Vervollständigung eines 2,5,6-Trimethyl-3,4-pyridinylen-Ringes)
Eine Mischung aus 3,8 g 2,5,6-Trimethylpyridin-3,4-dicarbonsäureanhyrid, 3,1 g Naphthalin-1,8-diamin und 20 g Phenol wird 30 Min unter Stickstoff auf 150°C erhitzt. Anschließend kühlt man auf 80°C, verdünnt mit 10 ml Methanol und saugt bei Raumtemperatur den gebildeten Niederschlag ab. Nach Methanolwäsche und Trocknen erhält man 4,8 g des Produktes der obigen Formel, welches Kunststoffe in rotstichig orangen hochechten Farbtönen gemäß der Färbebeispiele B) aus Beispiel 1 färbt.

### Beispiel 4

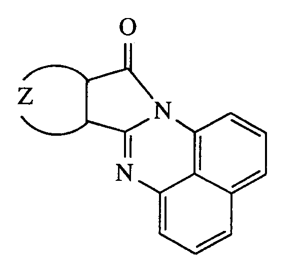

(Z = Rest zur Vervollständigung eines 6-Phenyl-3,4-pyridinylen-Ringes)
Eine Mischung aus 20 g Phenol, 3,1 g Naphthalin-1,8-diamin und 4,9 g 6-Phenylpyridin-3,4-dicarbonsäure wird in 30 min auf 150°C erhitzt und unter Abdestillieren des Reaktionswassers 90 min bei dieser Temperatur gehalten. Anschließend fällt man den Farbstoff mit 10 ml Methanol bei 60-80°C, saugt bei Raumtemperatur ab und wäscht mit Methanol nach.
Ausbeute: 1,4g; zur Reinigung kann aus Toluol umkristallisiert werden.

Der Farbstoff ergibt in Kunststoffen analog zu den Färbebeispielen B) in Beispiel 1 gefärbt, rote Färbungen mit hohem Echtheitsniveau.

### Beispiel 5

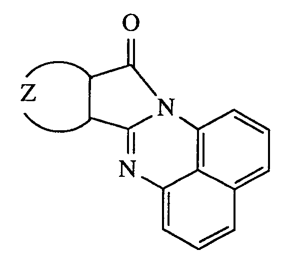

(Z = Rest zur Vervollständigung eines 2-Methyl-6-phenyl-3,4-pyridinylen-Ringes)
7,9 g Naphthalin-1,8-diamin werden bei 50°C in Phenol (50 g) vorgelegt und dann mit 12,9 g 2-Methyl-6-phenylpyridin-3,4-dicarbonsäure versetzt. Danach erhitzt man in 30 min auf 150°C und hält die Temperatur noch 30 min. Anschließend kühlt man auf 100°C ab, tropft 25 ml Methanol zu und kühlt dann auf Raumtemperatur ab. Der Niederschlag wird mit Methanol und heißem Wasser gewaschen und getrocknet.
Ausbeute: 16,5 g

Der Farbstoff färbt Kunststoffe gemäß der Färbebeispiele B) aus Beispeiel 1 in hochechten roten Farbtönen.

### Beispiel 6

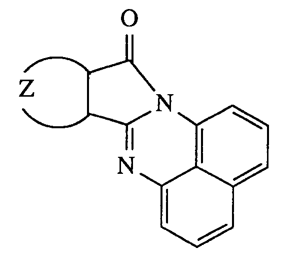

(Z = Rest zur Vervollständigung eines 2-Methyl-3,4-chinolinylen-Ringes)
Eine Mischung aus 6,0 g Chinolin-3,4-dicarbonsäureanhydrid, 4,8 g Naphthalin-1,8-diamin und 30 g Phenol wird unter Stickstoff 1 Stunde auf 135°C erhitzt. Anschließend fällt man das Produkt mit 25 ml Methanol bei 80°C und saugt den Niederschlag bei 40°C ab. Nach dem Waschen mit Methanol und Trocknen im Vakuum erhält man 7,8 g des Farbstoffes der obigen Formel als Mischung der beiden Isomeren.

In Kunststoffen analog zu den Färbebeispielen B) aus Beispiel 1 gefärbt liefert der Farbstoff rotviolette Färbungen mit guten Echtheiten.

### Beispiel 7

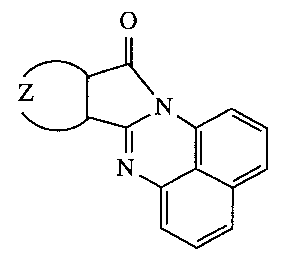

(Z = Rest zur Vervollständigung eines 2,3-Pyridinylen-Ringes)
15,8 Teile 1,8-Diamino-naphthalin werden in 70 Volumteilen Propionsäure unter Stickstoffatmosphäre gelöst. Man erwärmt auf 130°C und tropft unter Rühren 14,9 Teile Pyridin-2,3-dicarbonsäureanhydrid zügig ein. Das Reaktionsgemisch wird anschließend 5 Stunden bei 130°C gerührt. Nach dem Erkalten wird das ausgeschiedene Reaktionsgemisch abgesaugt und mit 50 Volumteilen Methanol und 100 Volumteilen Wasser gewaschen. Man erhält nach dem Trocknen bei 50°C im Vakuum 12,2 Teile des amorphen Isomerengemisches, das aus ca. 160 Volumteilen Dimethylformamid in orangen Balken kristallisiert werden kann.

Der Farbstoff färbt Kunststoffe gemäß dem Färbebeispiel B) aus Beispiel 1 in hochechten orangen Farbtönen.

## Patentansprüche

1. Farbstoffe der Formel 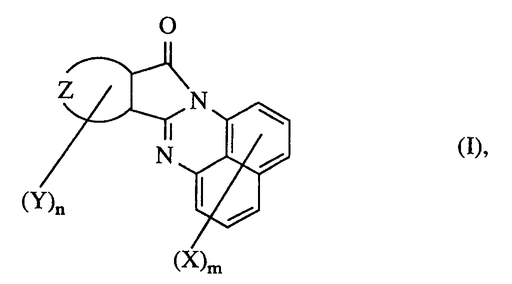 worin
Z ein Rest zur Vervollständigung eines 3,4- oder 2,3-Pyridinylen-, eines 4,5-Pyrimidinylen-, eines 3,4- oder 4,5-Pyridazinylen- oder eines 2,3-Pyrazinylen-Systems bedeutet,
X Alkyl, Halogen, Nitro, Chlorsulfonyl, Aryloxysulfonyl, Hydroxy, Alkoxy, Acyloxy, ein unsubstituiertes oder durch Alkyl oder Aryl substituiertes Aminosulfonyl, oder ein ankondensierter cycloaliphatischer oder heterocyclischer Ring ist,
Y Alkyl, Aryl, Halogen, Nitro, Hydroxy, Alkoxy, Acyloxy, Aryloxysulfonyl, Chlorsulfonyl, eine gegebenenfalls durch Acyl oder Alkyl substituierte Aminogruppe, ein gegebenenfalls durch Alkyl oder Aryl substituierter Aminosulfonylrest oder ein ankondensierter cycloaliphatischer oder aromatischer Ring ist,
m eine Zahl zwischen 0 und 6,
n eine Zahl zwischen 0 und 3 ist,
und für m > 1
X jeweils verschiedene oder gleiche der oben genannten Bedeutungen haben kann,
und für n > 1
Y jeweils verschiedene oder gleiche der oben genannten Bedeutungen haben kann.

2. Farbstoffe gemäß Anspruch 1, worin Z ein Rest zur Vervollständigung eines 3,4-Pyridinylensystems bedeutet.

3. Farbstoffe gemäß Anspruch 1, dadurch gekennzeichnet, daß
X für Chlor, Brom, -NO₂, -OCH₃, -OCH₂(C₆H₅), Chlorsulfonyl, -OH, -SO₂O(C₆H₅), -SO₂N(CH₃)₂, -SO₂NHCH₃, Methyl, Ethyl, n-Propyl, Isopropyl, n-Butyl, iso-Butyl, sek.-Butyl, tert.-Butyl oder ein vorzugsweise in 4,5-Stellung ankondensierter cycloaliphatischer 5-oder 6-Ring steht,
Y Methyl, Ethyl, n-Propyl, Isopropyl, n-Butyl, iso-Butyl, sek.-Butyl, tert.-Butyl, ein gegebenenfalls durch C₁-C₆-Alkoxy, C₁-C₆-Alkyl, Cl, Br oder F substituiertes Phenyl, ein ankondensierter cycloaliphatischer 5-, 6- oder 7-Ring oder ein ankondensierter Benzo-Ring ist,
m eine Zahl zwischen 0 und 4 ist und
n eine Zahl zwischen 0 und 3 ist.

4. Farbstoffe der Formel (I) gemäß Anspruch 1, dadurch gekennzeichnet, daß m gleich null ist.

5. Verfahren zur Herstellung der Farbstoffe (I) gemäß Anspruch 1, dadurch gekennzeichnet, daß man Dicarbonsäuren bzw. deren funktionelle Derivate der Formel (II) 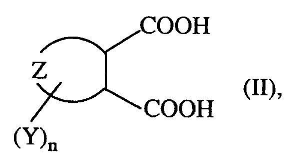 worin Z, Y und n die unter Anspruch 1 genannten Bedeutungen haben, mit Naphthalin-1,8-diaminen der Formel (III) 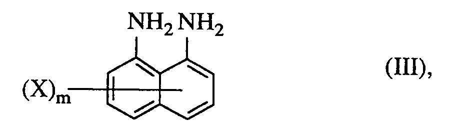 in der X und m die unter Anspruch 1 genannte Bedeutung haben, in der Schmelze oder in einem Lösungsmittel bei Temperaturen zwischen 20°C und 220°C, gegebenenfalls in Gegenwart eines sauren Katalysators und/oder unter Druck, umsetzt.

6. Verfahren gemäß Anspruch 5, dadurch gekennzeichnet, daß man Dicarbonsäuren der Formel (II) bzw. deren Anhydride verwendet, worin Z ein Rest zur Vervollständigung eines 3,4-Pyridinylensystems bedeutet.

7. Verfahren zum Massefärben von Kunststoffen, dadurch gekennzeichnet, daß ein Farbstoff der Ansprüche 1 bis 4 verwendet wird.

8. Verfahren gemäß Anspruch 7, dadurch gekennzeichnet, daß der Kunststoff ein Thermoplast ist.

9. Verfahren nach Anspruch 7 oder 8, dadurch gekennzeichnet, daß der zu färbende Kunststoff ein Vinylpolymeres, insbesondere Polystyrol, oder ein Polyester ist.

10. Kunststoffe, gefärbt nach wenigstens einem der Verfahren 7 bis 9.
